# EUROPEAN PATENT SPECIFICATION

(11) **EP 1 923 067 B1**
(45) Date of publication and mention of the grant of the patent: **13.04.2016**
(21) Application number: 06024004.1
(22) Date of filing: 20.11.2006
(51) Int. Cl.: A61K 36/48, A61K 36/906, A61P 3/10, A61P 3/04, A61K 131/00, A61K 125/00

(54) **Compositions containing phaseolus vulgaris extract and alpinia officinarum extract for the prevention and treatment of obesity and type II diabetes**
Zusammensetzungen, die Extrakte von Phaseolus vulgaris und Alpinia officinarum enthalten, zur Prävention und Behandlung von Fettleibigkeit und Typ II Diabetes
Compositions contenant un extrait de Phaseolus vulgaris et d'Alpinia officinarum pour le traitement de l'obésité et de diabète de type II

(43) Date of publication of application: 21.05.2008
(73) Proprietor: S.I.I.T. S.r.l.-Servizio Internazionale Imballaggi Termosaldanti, 20090 Trezzano sul Naviglio MI (IT)
(72) Inventor: Di Pierro, Francesco, 20090 Trezzano S/N (Milano) (IT)
(74) Representative: Minoja, Fabrizio

(56) References cited:
- EP-A2- 1 295 535
- WO-A-2006/039807
- JP-A- 5 255 100
- US-A1- 2005 186 278
- SHIN JI-EUN ET AL: "3-Methylethergalangin isolated from Alpinia officinarum inhibits pancreatic lipase." BIOLOGICAL AND PHARMACEUTICAL BULLETIN, vol. 26, no. 6, June 2003 (2003-06), pages 854-857, XP002428810 ISSN: 0918-6158
- PFISTER-HOTZ G: "PHYTOTHERAPIE UND STOFFWECHSEL 16. SCHWEIZERISCHE TAGUNG FUER PHYTOTHERAPIE (II) PHYTOTHERAPY AND METABOLISM. THE 16TH SWISS MEETING ON PHYTOTHERAPY (II)" ZEITSCHRIFT FUER PHYTOTHERAPIE, STUTTGART, DE, vol. 25, no. 5, 2004, pages 248-252, XP009058789
- AKHTAR ET AL: "Hypoglycaemic activity of Alpinia galanga rhizome and its extracts in rabbits" MEDICINAL & AROMATIC PLANTS ABSTRACTS, NATIONAL INSTITUTE OF SCIENCE COMMUNICATION AND INFORMATION, IN, vol. 25, no. 3, June 2003 (2003-06), XP018010613 ISSN: 0250-4367

## Description

### FIELD OF INVENTION

This invention relates to compositions containing *Phaseolus vulgaris* extract and *Alpinia officinarum* extract for the prevention and treatment of obesity and type II diabetes.

### PRIOR ART

The typical diet of the socio-economically developed countries leads to a significant increase in the incidence of disorders associated with overweight, obesity and type II diabetes.

Their common denominator, namely an excessive rise in the blood glucose curve, explains many of the consequences of these disorders.

Drastic changes in diet alone would obviously lead to a significant improvement in the situation, but drugs which can assist and support the desirable (but not always possible) dietary changes are still useful.

Eating complex carbohydrates (pasta, bread, rice, potatoes, etc.) is known to generate the production of pancreatic alpha-amylase, which is responsible for the digestion of glucose polymer starches consisting of 3 to 9 sub-units. Intestinal maltase and alpha-dextrinase cause the digestion of these short polymers with free glucose units, which generates the blood glucose curve. An increase in blood glucose produces the "insulin peak", which literally cleanses the blood of free glucose by sending it to the brain and storing it in the muscles and liver in the form of glycogen rosettes. As the muscles and liver are usually already full of these rosettes, the action of insulin leads to the transformation of free glucose into fat deposits (adipose tissue).

Alpha-amylase inhibitors able to prevent this cascade of events are known. Phaseolamin, a heat- and gastro-unstable protein of approximately 55 KD, obtained by extraction with hot water from the fruit of *Phaseolus vulgaris* (the kidney bean), is well known to be a powerful alpha-amylase inhibitor at very low molarities. Phaseolamin is available on the market with various degrees of specific activity, depending on the commercial source, and is present in various diet supplements and dietetic products.

As phaseolamin is a protein, it is broken down and consequently deactivated by the gastric juices. Gastroprotection of phaseolamin allows over 98% to be released into the enteric environment, and allows the inhibition of pancreatic alpha-amylase and blocking of the cascade of events that normally leads to a rise in the blood glucose curve.

JP05255100 teaches a lipase inhibitor containing as active ingredient a solvent of Alpinia officinarum rhizoma to contribute to control and prevention of overweight.

Italian patent publication no. ITMI20040313 filed by the Applicant, discloses the fact that gastroprotected phaseolamin is more active and allows a reduction in daily dose or the use of a phaseolamin whose specific activity is not particularly high. According to that patent application, gastroprotection is obtained with a coating of shellac (or another compatible polymer which is able to perform the same function and approved for nutritional use for regulatory purposes) which enables 98% of the enzyme to be released in active form into the enteric environment, thus allowing inhibition of pancreatic alpha-amylase (released in the stomach) and preventing the cascade of events that normally leads to a rise in the blood glucose curve.

*Alpinia officinarum,* also known as lesser galangal, is a plant belonging to the Zingiberaceae family originating from China, where it is used in traditional medicine as a digestive, antiemetic, carminative, antibacterial and anti-inflammatory agent. The active constituents present in the rhizome include galangol, galangin, prostaglandins, benzoic and oxalic cineolacids, starches and kaempferol.

The inhibiting action of a particular fraction of *Alpinia officinarum* extract on pancreatic lipase was recently demonstrated. In particular, the fraction soluble in ethyl acetate, enriched and with a standardised content of 3-methylethergalangin, the component responsible for inhibition of pancreatic lipase, with an IC₅₀ of around 1 mg/ml and consequently at a highly acceptable molarity, has been identified. This inhibition reduces the absorption of lipids and triglycerides. The plasma evaluations indicate the high statistical significance of this direct lipid-reducing effect on the triglycerides. However, this fraction, and the resulting lipase inhibition, does not produce a cholesterol-lowering effect, thus providing a further demonstration that it only has a direct, specific action on pancreatic lipase.

3-methylethergalangin, or an enriched fraction thereof, does not require gastroprotection, as the compound is not broken down by the gastric juices.

### DESCRIPTION OF THE INVENTION

It has now been discovered that the association of *Phaseolus vulgaris* extract with a standardised phaseolamin content and *Alpinia officinarum extract* with a standardised 3-methylethergalangin content is particularly effective for the prevention and treatment of obesity and type II diabetes.

This invention consequently relates to compositions containing *Phaseolus vulgaris extract* with a standardised phaseolamin content and *Alpinia officinarum* extract with a standardised 3-methylethergalangin content for the prevention and treatment of obesity and type II diabetes.

More particularly, the compositions according to the invention contain phaseolamin and 3-methylethergalangin in the ratio of 1:5.

The compositions to which this invention relates are in gastroprotected form, to prevent the breakdown of phaseolamin on contact with the gastric juices and to guarantee the stability of 3-methylethergalangin even at a pH of 1.

According to a preferred aspect, the compositions according to the invention will contain *Alpinia officinarum* extract in ethyl acetate, with a standardised 3-methylethergalangin content.

According to a preferred aspect, the compositions according to the invention will contain Indena phaseolamin standardised from 5 to 18% (with a phytohaemagglutinin content of between 0.01 and 0.06%). This phaseolamin will be gastroprotected according to the process described in Italian patent publication no. ITMI20040313.

The phaseolamin content of the compositions according to the invention will range between approx. 0.1 and approx. 1000 mg, preferably between 2 and 10 mg.

The 3-methylgalangin content of the compositions according to the invention will range between approx. 0.1 and approx. 500 mg, preferably between 1 and 100 mg.

The compositions according to the invention cause a reduction in the blood glucose peak and the postprandial lipid peak greater than that generated by the sum of the effects obtained after separate administration of the individual constituents of the association, apparently due to synergy between the individual constituents.

The compositions according to the invention will preferably be taken a few minutes before meals, to ensure that the product arrives when pancreatic secretion has begun and just before emptying of the stomach, with arrival of the food at the same level. This administration will reduce the absorption of free sugar, lipids and triglycerides, with a consequent calorie reduction and a reduced risk of obesity and diabetes.

The compositions according to the invention could be formulated suitably for oral administration, and will be prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use.

Reference Examples of formulations are set out below.

### REFERENCE EXAMPLE 1 - Gastroprotected tablets

| **INGREDIENT** | **mg** | **%** |
|---|---|---|
| Alpinia officinarum | 50.000 | 12.500 |
| Concentrated kidney bean protein | 30.000 | 7.500 |
| Dicalcium phosphate | 139.987 | 34.997 |
| Microcrystalline cellulose | 121.470 | 30.368 |
| Shellac | 15.000 | 3.750 |
| Croscarmellose sodium | 12.000 | 3.000 |
| Hydroxypropyl methylcellulose | 8.450 | 2.113 |
| Talc | 7.713 | 1.928 |
| E171 colouring | 2.831 | 0.708 |
| Triethyl citrate | 1.974 | 0.493 |
| Stearic acid | 1.300 | 0.325 |
| Ammonium carbonate | 1.012 | 0.253 |
| Vegetable magnesium stearate | 4.000 | 1.000 |
| Silicon dioxide | 4.000 | 1.000 |
| Yellow iron oxide | 0.263 | 0.066 |
| **TOTAL** | **400.00** | |

### REFERENCE EXAMPLE 2 - Mouth-soluble sachets

| **INGREDIENT** | **mg** | **%** |
|---|---|---|
| Alpinia officinarum | 50.000 | 2.77778 |
| Concentrated kidney bean protein | 30.000 | 1.66667 |
| Mono- and diglycerides of fatty acids | 100.000 | 5.55556 |
| Fructose | 528.000 | 29.3333 |
| Sorbitol | 524.000 | 29.1111 |
| Fruit oligosaccharides | 500.000 | 27.7778 |
| Flavouring | 50.000 | 2.77778 |
| Silicon dioxide | 15.000 | 0.83333 |
| Anhydrous citric acid | 3.000 | 0.16667 |
| **TOTAL** | **1800**.**00** | |

### REFERENCE EXAMPLE 3 - 0-shaped capsules

| **INGREDIENT** | **mg** | **%** |
|---|---|---|
| Alpinia officinarum | 50.000 | 12.500 |
| Concentrated kidney bean protein | 30.000 | 7.500 |
| Mono- and diglycerides of fatty acids | 100.000 | 25.000 |
| Microcrystalline cellulose | 58.000 | 14.500 |
| Dicalcium phosphate | 59.000 | 14.750 |
| Silicon dioxide | 4.000 | 1.000 |
| Magnesium stearate | 4.000 | 1.000 |
| | | |
| *Gelatin shell* | *95* | |
| **TOTAL** | **400.000** | |

## Claims

1. Compositions containing kidney bean extract with a standardised phaseolamin content, and *Alpinia officinarum* extract with a standardised 3-methylethergalangin content, for use in the prevention and treatment of obesity and type II diabetes.

2. Compositions for use as claimed in claim 1, in gastroprotected forms.

3. Compositions for use as claimed in claims 1 and 2, containing phaseolamin standardised to 18%, with a phytohaemagglutinin content of 0.06%.

4. Compositions for use as claimed in the preceding claims, containing *Alpinia officinarum* extract in ethyl acetate, with a standardised 3-methylethergalangin content.

5. Use of kidney bean extract with a standardised phaseolamin content, and *Alpinia officinarum* extract with a standardised 3-methylethergalangin content, to prepare compositions for the prevention and treatment of obesity and type II diabetes.

## Patentansprüche

1. Zusammensetzungen, enthaltend Kidneybohnen-Extrakt mit einem standardisierten Phaseolamin-Gehalt, und *Alpinia officinarum*-Extrakt mit einem standardisierten 3-Methylethergalangin-Gehalt, zur Verwendung bei der Vorbeugung und Behandlung von Fettsucht und Typ II-Diabetes.

2. Zusammensetzungen zur Verwendung wie in Anspruch 1 beansprucht in magensaftgeschützten Formen.

3. Zusammensetzungen für die Verwendung wie in den Ansprüchen 1 und 2 beansprucht, enthaltend auf 18 % standardisiertes Phaseolamin mit einem Phytohämagglutinin-Gehalt von 0,06 %.

4. Zusammensetzungen zur Verwendung wie in den vorstehenden Ansprüchen beansprucht, enthaltend *Alpinia officinarum*-Extrakt in Ethylacetat, mit einem standardisierten 3-Methylethergalangin-Gehalt.

5. Verwendung von Kidneybohnen-Extrakt mit einem standardisierten Phaseolamin-Gehalt und *Alpinia officinarum*-Extrakt mit einem standardisierten 3-Methylethergalangin-Gehalt, zur Herstellung von Zusammensetzungen zur Vorbeugung und Behandlung von Fettsucht und Typ II-Diabetes.

## Revendications

1. Compositions contenant de l'extrait de haricot commun à teneur normalisée en phaséolamine, et de l'extrait d*'Alpinia officinarum* à teneur normalisée en 3-méthyléthergalangine, pour utilisation dans la prévention et le traitement de l'obésité et du diabète de type II.

2. Compositions pour utilisation selon la revendication 1, sous formes gastroprotégées.

3. Compositions pour utilisation selon les revendications 1 et 2, contenant de la phaséolamine normalisée à 18 %, à teneur en phytohémagglutinine de 0,06 %.

4. Compositions pour utilisation selon les revendications précédentes, contenant de l'extrait d'*Alpinia officinarum* dans de l'acétate d'éthyle, à teneur normalisée en 3-méthyléthergalangine.

5. Utilisation d'extrait de haricot commun à teneur normalisée en phaséolamine, et d'extrait d'*Alpinia officinarum* à teneur normalisée en 3-méthyléthergalangine, pour préparer des compositions destinées à la prévention et au traitement de l'obésité et du diabète de type II.
